# EUROPEAN PATENT SPECIFICATION

(11) **EP 1 165 065 B1**
(45) Date of publication and mention of the grant of the patent: **07.05.2003**
(21) Application number: 00920520.4
(22) Date of filing: 21.03.2000
(51) Int. Cl.: A61K 31/198, A61K 9/20, A61K 9/36

(54) **SWALLOWABLE TABLETS WITH HIGH CONTENT OF N-ACETYLCYSTEINE**
SCHLUCKBARE TABLETTEN MIT EINEM HOHEN GEHALT AN N-ACETYLCYSTEIN
COMPRIMES A AVALER A TENEUR ELEVEE EN N-ACETYLCYSTEINE

(30) Priority: 06.04.1999 IT MI990704
(43) Date of publication of application: 02.01.2002
(73) Proprietor: ZAMBON GROUP S.p.A., 36100 Vicenza (IT)
(72) Inventor: CASTEGINI, Franco, I-36100 Vicenza (IT); GRASSANO, Alessandro, I-20052 Monza (IT); BARINA, Riccardo, I-30022 Ceggia (IT); ZULIANI, Italo, I-20052 Monza (IT); GURRIERI, Giovanni, I-37023 Grezzana (IT)
(74) Representative: Panossian, Stefano
(86) International application number: EP0002464
(87) International publication number: WO00059500

(56) References cited:
- EP-A- 0 315 249
- EP-A- 0 481 294
- WO-A-96/35452

## Description

The present invention relates to swallowable tablets with high content of N-acetylcysteine. N-acetylcysteine (NAC) is a compound known for time and mainly used in therapy as mucolytic and expectorant (The Merck Index, XII ed., page 16, no. 89).

Usually NAC is administered by topical route or by oral route in the form of a granulate or tablet.

For the oral solid administration, presently NAC is formulated only at the maximum dose of 200 mg in chewable tablets to be dissolved in the mouth. This implies the need to mask the taste as well as the smell of this active ingredient, known to be not very pleasant because of the presence of a sulphur group in the molecule.

Just for the need to use a high number and amount of excipients to mask the taste and the smell, there are on the market neither oral NAC formulations containing more than 65% by weight of active ingredient nor NAC formulations as swallowable tablets with a dose higher than 200 mg/tablet.

The formulation of tablets for the oral administration containing a high percentage of active ingredient is generally difficult.

US patent 4,908,210 (Eastman Kodak Company) claims a compressible powder containing 0.5-5% by weight of a specific mixture of lubricants (monoglycerides, propylene-glycol monoesters, a salt of a fatty acid ester of lactic acid) so to give finished tablets containing a percentage of active ingredient higher than 80%. Such a powder shows economic drawbacks since some excipients of the lubricant mixture are particularly expensive.

US patent 5,501,861 (Takeda Chemical Industry Ltd) illustrates fast dissolving tablets containing the active ingredient in the percentage of 0.05-90% by weight of the semifinished product and a hydrosoluble carbohydrate in a percentage of 30-80% by weight. However, this document underlines that the use of 30-70% by weight of active ingredient is preferred to obtain a tablet with a high dosage. In fact, the examples of final formulation never contain percentages of active ingredient higher than 50%.

US patent 5,401,514 (Spirig AG) claims an oral solid formulation of at least 50% NAC or carboxy-methyl-cysteine by weight in admixture with at least a cellulose, a soluble sugar, a sweetener and a flavour. The same formulation is claimed with a NAC content of 100 mg and 200 mg. The content of active ingredient in the exemplified formulations reaches 65% by weight at the maximum.

A swallowable tablet with a high content of NAC has been now found.

Therefore, the present invention relates to a swallowable tablet containing 80-95% NAC by weight, 0.5-4% by weight with respect to NAC of a binder and further pharmaceutically acceptable excipients.

Preferably, the swallowable tablet object of the present invention contains 80-95% NAC by weight, 1-3% binder by weight, 2.5-14% diluent by weight, 1-4.5% disintegrant by weight, 0.1-1.5% lubricant by weight, optionally in the presence of a flowing agent and of a film-coating layer.

Still more preferably, the swallowable tablet object of the present invention contains 80-90% NAC by weight, 2-3% binder by weight, 3-14% diluent by weight, 1-4.5% disintegrant by weight, 0.1-1% lubricant by weight, 0.1-1% flowing agent and optionally a film-coating layer equal to or lower than 4% by weight.

Examples of binders according to the present invention are linear polyvinylpyrrolidone, sodium carboxymethylcellulose, ethylcellulose, methylcellulose, liquid glucose, gelatine and hydroxypropylcellulose.

Preferably linear polyvinylpirrolidone is used.

Examples of lubricants according to the present invention are magnesium stearate, sodium stearyl fumarate, sodium benzoate and polyethylene glycol.

Magnesium stearate is preferably used.

Examples of diluents according to the present invention are carbohydrates, such as lactose and saccharose, microcrystalline cellulose and derivatives thereof, inorganic salts, such as dibasic calcium phosphate and sodium bicarbonate, polyalcohols, such as sorbitol, mannitol and xylitol, and mixtures thereof.

Microcrystalline cellulose and its derivatives or mixtures thereof are preferably used.

Examples of disintegrants according to the present invention are cross-linked polyvinylpyrrolidone, sodium croscarmellose. sodium carboxymethyl starch, starch, pregelatinized starch and microcrystalline cellulose.

Cross-linked polyvinylpyrrolidone and sodium croscarmellose are preferably used.

When present, the preferably used flowing agent is colloidal silica.

The tablet object of the invention is prepared by wet granulation of the active ingredient with a binder and optionally all or part of the estimated amount of diluent and/or of the lubricant. The granulate is then mixed and compressed with the other excipients and the resultant tablet optionally undergoes a film-coating procedure.

This optional film-coating is carried out according to conventional techniques by preferably using cellulose acetate phthalate, ethylcellulose, hydroxypropylcellulose, hydroxypropylmethylcellulose and metacrylic acid co-polymers, as coating agents, in admixture with opacifiers, plasticizers and, optionally, dyes and sweetening agents.

Preferably the preparation of the swallowable tablet object of the present invention is carried out by wet granulation of NAC with a binder. The resultant granulate is then mixed with the remaining excipients and compressed.

The use of such a low amount of excipients makes possible the preparation of swallowable tablets containing a high dose of NAC, while keeping the final size of the tablet within acceptable values, that is suitable for the swallowing.

A particularly preferred feature of the present invention is therefore represented by the preparation of tablets containing a dose of NAC equal to or higher than 400 mg, more preferably equal to 600 mg.

The tablets containing 600 mg NAC, according to the present invention, have a weight from 630 mg to 750 mg. For the preparation of tablets having this weight, conventional moulds such as, for example, convex tablets with 12-mm diameter or capsule-shaped tablets with sizes 18.16 x 7.41 mm, commonly used for swallowable tablets, can be used.

Furthermore, the tablets object of the present invention show physical characteristics which fulfil the requirements imposed by the Official Pharmacopoeias.

For example, the hardness of the tablets will be generally from 6 to 14 Kp. the friability from 0.05 to 0.7% and the disintegration time lower than 15 minutes for the non-film coated tablets.

In order to better illustrate the present invention the following examples are now given.

### Example 1

### Preparation of the granulates

By using a wet granulation procedure in a rotogranulator, the following granulates were prepared (% by weight):

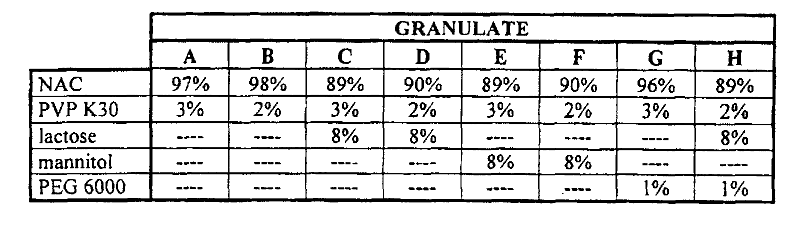

The granulates (2 Kg each batch) were dried, screened and used for the preparation of the tablets according to what reported in the following examples.

### Example 2

Granulate A (2 Kg) was mixed with microcrystalline cellulose, sodium bicarbonate, PVP CL and magnesium stearate. The mixture was compressed to obtain tablets having the following composition expressed as mg/tablet (% by weight):

| | | |
|---|---|---|
| NAC | 600.0 mg | (84.4%) |
| PVP K30 | 18.0 mg | (2.5%) |
| microcrystalline cellulose | 50.8 mg | (7.1%) |
| sodium bicarbonate | 20.0 mg | (2.8%) |
| PVP CL | 16.0 mg | (2.3%) |
| magnesium stearate | 6.2 mg | (0.9%) |
| total weight | 711.0 mg | |

### Example 3

Granulate A (2 Kg) was mixed with microcrystalline cellulose, PVP CL and magnesium stearate. The mixture was compressed to obtain tablets having the following composition expressed as mg/tablet (% by weight):

| | | |
|---|---|---|
| NAC | 600.0 mg | (83.8%) |
| PVP K30 | 18.0 mg | (2.5%) |
| microcrystalline cellulose | 75.8 mg | (10.6%) |
| PVP CL | 16.0 mg | (2.2%) |
| magnesium stearate | 6.2 mg | (0.9%) |
| total weight | 716.0 mg | |

### Example 4

Granulate A (2 Kg) was mixed with microcrystalline cellulose, PVP CL, magnesium stearate and colloidal silica. The mixture was compressed to obtain tablets having the following composition expressed as mg/tablet (% by weight):

| | | |
|---|---|---|
| NAC | 600.0 mg | (83.9%) |
| PVP K30 | 18.0 mg | (2.5%) |
| microcrystalline cellulose | 60.0 mg | (8.4%) |
| PVP CL | 30.0 mg | (4.2%) |
| magnesium stearate | 3.5 mg | (0.5%) |
| colloidal silica | 3.5 mg | (0.5%) |
| total weight | 715.0 mg | |

### Example 5

Granulate A (2 Kg) was mixed with dibasic calcium phosphate, PVP CL, magnesium stearate and colloidal silica. The mixture was compressed to obtain tablets having the following composition expressed as mg/tablet (% by weight):

| | | |
|---|---|---|
| NAC | 600.0 mg | (83.8%) |
| PVP K30 | 18.0 mg | (2.5%) |
| dibasic calcium phosphate | 60.8 mg | (8.5%) |
| PVP CL | 30.0 mg | (4.2%) |
| magnesium stearate | 3.6 mg | (0.5%) |
| colloidal silica | 3.6 mg | (0.5%) |
| total weight | 716.0 mg | |

### Example 6

Granulate A (2 Kg) was mixed with lactose, PVP CL, magnesium stearate and colloidal silica. The mixture was compressed to obtain tablets having the following composition expressed as mg/tablet (% by weight):

| | | |
|---|---|---|
| NAC | 600.0 mg | (83.33%) |
| PVP K30 | 18.0 mg | (2.50%) |
| lactose | 75.0 mg | (10.42%) |
| PVP CL | 16.0 mg | (2.22%) |
| magnesium stearate | 6.4 mg | (0.89%) |
| colloidal silica | 4.6 mg | (0.64%) |
| total weight | 720.0 mg | |

### Example 7

Granulate A (2 Kg) was mixed with microcrystalline cellulose, sodium croscarmellose and magnesium stearate. The mixture was compressed to obtain tablets having the following composition expressed as mg/tablet (% by weight):

| | | |
|---|---|---|
| NAC | 600.0 mg | (85.7%) |
| PVP K30 | 18.0 mg | (2.6%) |
| microcrystalline cellulose | 45.8 mg | (6.5%) |
| sodium croscarmellose | 30.0 mg | (4.3%) |
| magnesium stearate | 6.2 mg | (0.9%) |
| total weight | 700.0 mg | |

### Example 8

Granulate A (2 Kg) was mixed with microcrystalline cellulose, PVP CL and magnesium stearate. The mixture was compressed to obtain tablets having the following composition expressed as mg/tablet (% by weight):

| | | |
|---|---|---|
| NAC | 600.0 mg | (85.7%) |
| PVP K30 | 18.0 mg | (2.6%) |
| microcrystalline cellulose | 45.8 mg | (6.5%) |
| PVP CL | 30.0 mg | (4.3%) |
| magnesium stearate | 6.2 mg | (0.9%) |
| total weight | 700.0 mg | |

### Example 9

Granulate C (2 Kg) was mixed with PVP CL, magnesium stearate and colloidal silica. The mixture was compressed to obtain tablets having the following composition expressed as me/tablet (% by weight).

| | | |
|---|---|---|
| NAC | 600.0 mg | (84.4%) |
| PVP K30 | 20.0 mg | (2.8%) |
| lactose | 54.0 mg | (7.6%) |
| PVP CL | 30.0 mg | (4.2%) |
| magnesium stearate | 4.0 mg | (0.6%) |
| colloidal silica | 3.0 mg | (0.4%) |
| total weight | 711.0 mg | |

### Example 10

Granulate D (2 Kg) was mixed with microcrystalline cellulose, PVP CL, magnesium stearate and colloidal silica. The mixture was compressed to obtain tablets having the following composition expressed as mg/tablet (% by weight):

| | | |
|---|---|---|
| NAC | 600.0 mg | (82.6%) |
| PVP K30 | 15.0 mg | (2.1%) |
| lactose | 54.0 mg | (7.4%) |
| microcrystalline cellulose | 30.0 mg | (4.1%) |
| PVP CL | 20.0 mg | (2.8%) |
| magnesium stearate | 4.0 mg | (0.6%) |
| colloidal silica | 3.0 mg | (0.4%) |
| total weight | 726.0 mg | |

### Example 11

Granulate E (2 Kg) was mixed with microcrystalline cellulose, PVP CL, magnesium stearate and colloidal silica. The mixture was compressed to obtain tablets having the following composition expressed as mg/tablet (% by weight):

| | | |
|---|---|---|
| NAC | 600.0 mg | (81.52%) |
| PVP K30 | 20.0 mg | (2.72%) |
| mannitol | 54.0 mg | (7.34%) |
| microcrystalline cellulose | 30.0 mg | (4.08%) |
| PVP CL | 25.0 mg | (3.40%) |
| magnesium stearate | 4.0 mg | (0.54%) |
| colloidal silica | 3.0 mg | (0.40%) |
| total weight | 736.0 mg | |

### Example 12

Granulate F (2 Kg) was mixed with dibasic calcium phosphate, PVP CL, magnesium stearate and colloidal silica. The mixture was compressed to obtain tablets having the following composition expressed as mg/tablet (% by weight):

| | | |
|---|---|---|
| NAC | 600.0 mg | (81.52%) |
| PVP K30 | 15.0 mg | (2.04%) |
| mannitol | 54.0 mg | (7.34%) |
| dibasic calcium phosphate | 40.0 mg | (5.44%) |
| PVP CL | 20.0 mg | (2.72%) |
| magnesium stearate | 4.0 mg | (0.54%) |
| colloidal silica | 3.0 mg | (0.40%) |
| total weight | 736.0 mg | |

### Example 13

Granulate G (2 Kg) was mixed with microcrystalline cellulose, sodium bicarbonate, PVP CL and magnesium stearate. The mixture was compressed to obtain tablets having the following composition expressed as mg/tablet (% by weight):

| | | |
|---|---|---|
| NAC | 600.0 mg | (84.39%) |
| PVP K30 | 18.0 mg | (2.53%) |
| PEG 6000 | 6.0 mg | (0.84%) |
| microcrystalline cellulose | 50.8 mg | (7.15%) |
| sodium bicarbonate | 14.0 mg | (1.97%) |
| PVP CL | 16.0 mg | (2.25%) |
| magnesium stearate | 6.2 mg | (0.87%) |
| total weight | 711.0 mg | |

### Example 14

Granulate G (2 Kg) was mixed with microcrystalline cellulose, colloidal silica. PVP CL and magnesium stearate. The mixture was compressed to obtain tablets having the following composition expressed as mg/tablet (% by weight):

| | | |
|---|---|---|
| NAC | 600.0 mg | (83.22%) |
| PVP K30 | 18.0 mg | (2.49%) |
| PEG 6000 | 6.0 mg | (0.83%) |
| PVPCL | 30.0 mg | (4.16%) |
| microcrystalline cellulose | 60.0 mg | (8.32%) |
| colloidal silica | 3.5 mg | (0.49%) |
| magnesium stearate | 3.5 mg | (0.49%) |
| total weight | 721.0 mg | |

### Example 15

Granulate G (2 Kg) was mixed with microcrystalline cellulose, colloidal silica, PVP CL and magnesium stearate. The mixture was compressed to obtain tablets having the following composition expressed as mg/tablet (% by weight):

| | | |
|---|---|---|
| NAC | 400.0 mg | (83.22%) |
| PVP K30 | 12.0 mg | (2.49%) |
| PEG 6000 | 4.0 mg | (0.83%) |
| PVP CL | 20.0 mg | (4.16%) |
| microcrystalline cellulose | 40.0 mg | (8.32%) |
| colloidal silica | 2.33 mg | (0.49%) |
| magnesium stearate | 2.33 mg | (0.49%) |
| total weight | 480.66 mg | |

### Example 16

Granulate G (2 Kg) was mixed with microcrystalline cellulose, colloidal silica, PVP CL and magnesium stearate. The mixture was compressed to obtain tablets having the following composition expressed as mg/tablet (% by weight):

| | | |
|---|---|---|
| NAC | 200.0 mg | (83.22%) |
| PVP K30 | 6.0 mg | (2.49%) |
| PEG 6000 | 2.0 mg | (0.83%) |
| PVP CL | 10.0 mg | (4.16%) |
| microcrystalline cellulose | 20.0 mg | (8.32%) |
| colloidal silica | 1.16 mg | (0.49%) |
| magnesium stearate | 1.16 mg | (0.49%) |
| total weight | 240.32 mg | |

### Example 17

Granulate A (2 Kg) was mixed with PVP CL, microcrystalline cellulose, colloidal silica and magnesium stearate. The mixture was compressed to obtain tablets having the following composition expressed as mg/tablet (% by weight):

| | | |
|---|---|---|
| NAC | 400.0 mg | (83.92%) |
| PVP K30 | 12.0 mg | (2.52%) |
| PVP CL | 20.0 mg | (4.19%) |
| microcrystalline cellulose | 40.0 mg | (8.39%) |
| colloidal silica | 2.33 mg | (0.49%) |
| magnesium stearate | 2.33 mg | (0.49%) |
| total weight | 476.66 mg | |

### Example 18

Granulate A (2 Kg) was mixed with PVP CL, microcrystalline cellulose, colloidal silica and magnesium stearate. The mixture was compressed to obtain tablets having the following composition expressed as mg/tablet (% by weight):

| | | |
|---|---|---|
| NAC | 200.0 mg | (83.92%) |
| PVP K30 | 6.0 mg | (2.52%) |
| PVP CL | 10.0 mg | (4.19%) |
| microcrystalline cellulose | 20.0 mg | (8.39%) |
| colloidal silica | 1.16 mg | (0.49%) |
| magnesium stearate | 1.16 mg | (0.49%) |
| total weight | 238.32 mg | |

### Example 19

The tablets prepared as described in example 4 were coated with a mixture of hydroxypropylmethylcellulose, titanium dioxide and PEG 6000.

The mixture was suspended in water (suspension at 10-15%) and sprayed into a coating pan. The resultant film-coated tablets have the following composition expressed as mg/tablet (% by weight):

| | | |
|---|---|---|
| NAC | 600.0 mg | (82.4%) |
| PVP K30 | 18.0 mg | (2.5%) |
| microcrystalline cellulose | 60.0 mg | (8.2%) |
| PVP CL | 30.0 mg | (4.1%) |
| magnesium stearate | 3.5 mg | (0.5%) |
| colloidal silica | 3.5 mg | (0.5%) |
| HPMC | 5.0 mg | (0.7%) |
| titanium dioxide | 5.0 mg | (0.7%) |
| PEG 6000 | 3.0 mg | (0.4%) |
| total weight | 728.0 mg | |

### Example 20

The tablets prepared as described in example 4 were coated with a mixture of metacrylic acid copolymer, titanium dioxide and polysorbate 80.

The mixture was suspended in water (suspension at 10-15%) and sprayed into a coating ban.

The resultant film-coated tablets have the following composition expressed as mg/tablet (% by weight):

| | | |
|---|---|---|
| NAC | 600.0 mg | (82.27%) |
| PVP K30 | 18.0 mg | (2.47%) |
| microcrystalline cellulose | 60.0 mg | (8.23%) |
| PVP CL | 30.0 mg | (4.11%) |
| magnesium stearate | 3.5 mg | (0.48%) |
| colloidal silica | 3.5 mg | (0.48%) |
| metacrylic acid copolymer | 9.2 mg | (1.26%) |
| titanium dioxide | 3.3 mg | (0.45%) |
| polysorbate 80 | 1.8 mg | (0.25%) |
| total weight | 729.3 mg | |

### Example 21

The tablets prepared as described in example 4 were coated with a mixture of polyvinylalcohol, titanium dioxide, talc, soya lecithin and xanthan gum, commercially available as Opadry OY-B-28920.

The mixture was suspended in water and sprayed into a coating pan. The resultant film-coated tablets (12 mm diameter) have the following composition expressed as mg/tablet (% by weight):

| | | |
|---|---|---|
| NAC | 600.0 mg | (80.7%) |
| PVP K30 | 18.0 mg | (2.42%) |
| PVP CL | 30.0 mg | (4.03%) |
| microcrystalline cellulose | 60.0 mg | (8.06%) |
| colloidal silica | 3.5 mg | (0.47%) |
| magnesium stearate | 3.5 mg | (0.47%) |
| Opadry OY-B-28920 | 28.6 mg | (3.85%) |
| total weight | 743.6 mg | |

### Example 22

The tablets prepared as described in example 14 were coated with a mixture of polyvinylalcohol, titanium dioxide, talc, soya lecithin and xanthan gum, commercially available as Opadry OY-B-28920.

The mixture was suspended in water and sprayed into a coating pan. The resultant film-coated tablets (12 mm diameter) have the following composition expressed as mg/tablet (% by weight):

| | | |
|---|---|---|
| NAC | 600.0 mg | (80.0%) |
| PVP K30 | 18.0 mg | (2.4%) |
| PEG 6000 | 6.0 mg | (0.8%) |
| PVP CL | 30.0 mg | (4.0%) |
| microcrystalline cellulose | 60.0 mg | (8.0%) |
| colloidal silica | 3.5 mg | (0.47%) |
| magnesium stearate | 3.5 mg | (0.47%) |
| Opadry OY-B-28920 | 28.84 mg | (3.86%) |
| total weight | 749.84 mg | |

### Example 23

The tablets prepared as described in example 15 were coated with a mixture of polyvinylalcohol, titanium dioxide, talc, soya lecithin and xanthan gum, commercially available as Opadry OY-B-28920.

The mixture was suspended in water and sprayed into a coating pan. The resultant film-coated tablets (10 mm diameter) have the following composition expressed as mg/tablet (% by weight)

| | | |
|---|---|---|
| NAC | 400.0 mg | (80.0%) |
| PVP K30 | 12.0 mg | (2.4%) |
| PEG 6000 | 4.0 mg | (0.8%) |
| PVP CL | 20.0 mg | (4.0%) |
| microcrystalline cellulose | 40.0 mg | (8.0%) |
| colloidal silica | 2.33 mg | (0.47%) |
| magnesium stearate | 2.33 mg | (0.47%) |
| Opadry OY-B-28920 | 19.24 mg | (3.86%) |
| total weight | 499.9 mg | |

### Example 24

The tablets prepared as described in example 16 were coated with a mixture of polyvinylalcohol, titanium dioxide, talc, soya lecithin and xanthan gum, commercially available as Opadry OY-B-28920.

The mixture was suspended in water and sprayed into a coating pan. The resultant film-coated tablets (8 mm diameter) have the following composition expressed as mg/tablet (% by weight):

| | | |
|---|---|---|
| NAC | 200.0 mg | (80.0%) |
| PVP K30 | 6.0 mg | (2.4%) |
| PEG 6000 | 2.0 mg | (0.8%) |
| PVP CL | 10.0 mg | (4.0%) |
| microcrystalline cellulose | 20.0 mg | (8.0%) |
| colloidal silica | 1.16 mg | (0.47%) |
| magnesium stearate | 1.16 mg | (0.47%) |
| Opadry OY-B-28920 | 9.58 mg | (3.86%) |
| total weight | 249.9 mg | |

### Example 25

The tablets prepared as described in example 17 were coated with a mixture of polyvinylalcohol, titanium dioxide, talc, soya lecithin and xanthan gum, commercially available as Opadry OY-B-28920.

The mixture was suspended in water and sprayed into a coating pan. The resultant film-coated tablets (10 mm diameter) have the following composition expressed as mg/tablet (% by weight):

| | | |
|---|---|---|
| NAC | 400.0 mg | (80.7%) |
| PVP K30 | 12.0 mg | (2.42%) |
| PVP CL | 20.0 mg | (4.03%) |
| microcrystalline cellulose | 40.0 mg | (8.06%) |
| colloidal silica | 2.33 mg | (0.47%) |
| magnesium stearate | 2.33 mg | (0.47%) |
| Opadry OY-B-28920 | 19.04 mg | (3.85%) |
| total weight | 495.7 mg | |

### Example 26

The tablets prepared as described in example 18 were coated with a mixture of polyvinylalcohol, titanium dioxide, talc, soya lecithin and xanthan gum, commercially available as Opadry OY-B-28920.

The mixture was suspended in water and sprayed into a coating pan. The resultant film-coated tablets (8 mm diameter) have the following composition expressed as mg/tablet (% by weight):

| | | |
|---|---|---|
| NAC | 200.0 mg | (80.7%) |
| PVP K30 | 6.0 mg | (2.42%) |
| PVP CL | 10.0 mg | (4.03%) |
| microcrystalline cellulose | 20.0 mg | (8.06%) |
| colloidal silica | 1.16 mg | (0.47%) |
| magnesium stearate | 1.16 mg | (0.47%) |
| Opadry OY-B-28920 | 9.53 mg | (3.85%) |
| total weight | 247.85 mg | |

### Example 27

The tablets prepared as described in example 4 were coated with a mixture of polyvinylalcohol, titanium dioxide, talc, soya lecithin and xanthan gum, commercially available as Opadry OY-B-28920, added with sodium saccharin.

The mixture was suspended in water and sprayed into a coating pan. The resultant film-coated tablets have the following composition expressed as mg/tablet (% by weight):

| | | |
|---|---|---|
| NAC | 600.0 mg | (80.6%) |
| PVP K30 | 18.0 mg | (2.4%) |
| PVP CL | 30.0 mg | (4.0%) |
| microcrystalline cellulose | 60.0 mg | (8.0%) |
| colloidal silica | 3.5 mg | (0.5%) |
| magnesium stearate | 3.5 mg | (0.5%) |
| Opadry OY-B-28920 | 28.6 mg | (3.8%) |
| sodium saccharin | 1.4 mg | (0.2%) |
| total weight | 745 mg | |

By working in a similar way, tablets having a lower content of sodium saccharin in the film-coating mixture, 1.0 mg and 0.5 mg respectively, were prepared.

## Claims

1. A swallowable tablet containing 80-95% N-acetylcysteine by weight, 0.5-4% by weight with respect to N-acetylcysteine of a binder and further pharmaceutically acceptable excipients.

2. A swallowable tablet according to claim 1 containing 80-95% N-acetylcysteine by weight, 1-3% binder by weight, 2.5-14% diluent by weight, 1-4.5% disintegrant by weight, 0.1-1.5% lubricant by weight optionally in the presence of a flowing agent and of a film-coating layer.

3. A swallowable tablet according to claim 1 or 2 containing 80-90% N-acetylcysteine by weight, 2-3% binder by weight, 3-14% diluent by weight, 1-4.5% disintegrant by weight, 0.1-1% lubricant by weight, 0.1-1% flowing agent and optionally a film-coating layer equal to or lower than 4% by weight.

4. A tablet according to one of the preceding claims wherein the binder is selected among linear polyvinylpyrrolidone, sodium carboxymethylcellulose, ethylcellulose, methylcellulose, liquid glucose, gelatine and hydroxypropylcellulose.

5. A tablet according to claim 4 wherein the binder is linear polyvinylpirrolidone.

6. A tablet according to one of the preceding claims wherein the lubricant is selected among magnesium stearate, sodium stearyl fumarate, sodium benzoate and polyethylene glycol.

7. A tablet according to claim 6 wherein the lubricant is magnesium stearate.

8. A tablet according to one of the preceding claims wherein the diluent is selected among carbohydrates, microcrystalline cellulose and derivatives thereof, inorganic salts, polyalcohols, and mixtures thereof.

9. A tablet according to claim 8 wherein the diluent is microcrystalline cellulose, its derivatives or mixtures thereof.

10. A tablet according to one of the preceding claims wherein the disintegrant is selected among cross-linked polyvinylpyrrolidone, sodium croscarmellose, sodium carboxymethyl starch, starch, pregelatinized starch and microcrystalline cellulose.

11. A tablet according to claim 10 wherein the disintegrant is cross-linked polyvinylpyrrolidone or sodium croscarmellose.

12. A tablet according to one of the preceding claims containing colloidal silica as flowing agent.

13. A process for the preparation of a swallowable tablet according to one of the preceding claims which comprises the wet granulation of N-acetylcysteine with a binder and optionally all or a part of the estimated amount of diluent, the mixing of the granulate with the remaining excipients, the compression and the optional film-coating of the tablet.

## Patentansprüche

1. Schluckbare Tablette, enthaltend 80-95 Gew.-% N-Acetylcystein, 0,5-4 Gew.-% bezogen auf N-Acetylcystein eines Bindemittels und ferner pharmazeutisch akzeptable Hilfsstoffe.

2. Schluckbare Tablette nach Anspruch 1, enthaltend 80-95 Gew.-% N-Acetylcystein, 1-3 Gew.-% Bindemittel, 2,5-14 Gew.-% Verdünnungsmittel, 1-4,5 Gew.-% Abbaumittel, 0,1-1,5 Gew.-% Gleitmittel, gegebenenfalls in Gegenwart eines Fließmittels und einer Filmüberzugsschicht.

3. Schluckbare Tablette nach Anspruch 1 oder 2, enthaltend 80-90 Gew.-% N-Acetylcystein, 2-3 Gew.-% Bindemittel, 3-14 Gew.-% Verdünnungsmittel, 1-4,5 Gew.-% Abbaumittel, 0,1-1 Gew.-% Gleitmittel, 0,1-1 Gew.-% Fließmittel und gegebenenfalls eine Filmüberzugsschicht von gleich oder weniger als 4 Gew.-%.

4. Tablette nach einem der vorherigen Ansprüche, wobei das Bindemittel ausgewählt ist aus linearem Polyvinylpyrrolidon, Natriumcarboxymethylcellulose, Etylcellulose, Methylcellulose, flüssiger Glucose, Gelatine und Hydroxypropylcellulose.

5. Tablette nach Anspruch 4, wobei das Bindemittel lineares Polyvinylpyrrolidon ist.

6. Tablette nach einem der vorherigen Ansprüche, wobei das Gleitmittel ausgewählt ist aus Magnesiumstearat, Natriumstearylfumarat, Natriumbenzoat und Polyethylenglykol.

7. Tablette nach Anspruch 6, wobei das Gleitmittel Magnesiumstearat ist.

8. Tablette nach einem der vorherigen Ansprüche, wobei das Verdünnungsmittel ausgewählt ist aus Kohlehydraten, mikrokristalliner Cellulose und Derivaten davon, anorganischen Salzen, Polyalkoholen und Mischungen davon.

9. Tablette nach Anspruch 8, wobei das Verdünnungsmittel mikrokristalline Cellulose, ihre Derivate oder Mischungen davon ist.

10. Tablette nach einem der vorherigen Ansprüche, wobei das Abbaumittel ausgewählt ist aus vernetztem Polyvinylpyrrolidon, Natriumcroscarmellose, Natriumcarboxymethylstärke, Stärke, vorgelierter Stärke, und mikrokristalliner Cellulose.

11. Tablette nach Anspruch 10, wobei das Abbaumittel vernetztes Polyvinylpyrrolidon oder Natriumcroscarmellose ist.

12. Tablette nach einem der vorherigen Ansprüche, welche kolloidales Siliciumdioxid als Fließmittel enthält.

13. Verfahren zur Herstellung einer schluckbare Tablette gemäß einem der vorherigen Ansprüche, welches das Naßgranulieren von N-Acetylcystein mit einem Bindemittel und gegebenenfalls der ganzen oder einem Teil der veranschlagten Menge an Verdünnungsmittel, Mischen des Granulates mit den verbleibenden Hilfsstoffen, Kompression und gegebenenfalls Filmbeschichtung der Tablette umfaßt.

## Revendications

1. Comprimé à avaler contenant 80-95 % en poids de N-acétylcystéine, 0,5 - 4 % en poids par rapport à la N-acétylcystéine d'un liant et d'autres excipients pharmaceutiquement acceptables.

2. Comprimé à avaler selon la revendication 1, contenant 80-95 % en poids de N-acétylcystéine, 1 - 3 % en poids de liant, 2,5 - 14 % en poids de diluant, 1 - 4,5 % en poids de désintégrant, 0,1 - 1,5 % en poids de lubrifiant, éventuellement en présence d'un agent d'écoulement et d'une couche pélliculante.

3. Comprimé à avaler selon la revendication 1 ou 2, contenant 80-90 % en poids de N-acétylcystéine, 2 - 3% en poids de liant, 3 - 14 % en poids de diluant, 1 - 4,5 % en poids de désintégrant, 0,1 - 1 % en poids de lubrifiant, 0,1 - 1 % en poids d'agent d'écoulement et éventuellement une couche pélliculante égale ou inférieure à 4 % en poids.

4. Comprimé selon l'une quelconque des revendications précédentes, dans lequel le liant est choisi parmi la polyvinylpyrrolidone linéaire, la carboxyméthylcellulose de sodium, l'éthylcellulose, la méthylcellulose, le glucose liquide, la gélatine et l'hydroxypropylcellulose.

5. Comprimé selon la revendication 4, dans laquelle le liant est la polyvinylpyrrolidone linéaire.

6. Comprimé selon l'une quelconque des revendications précédentes dans lequel le lubrifiant est choisi parmi le stéarate de magnésium, le stéaryl fumarate de sodium, le benzoate de sodium et le polyéthylène glycol.

7. Comprimé selon la revendication 6, dans lequel le lubrifiant est le stéarate de magnésium.

8. Comprimé selon l'une quelconque des revendications précédentes dans lequel le diluant est choisi parmi les carbohydrates, la cellulose microcristalline et des dérivés de celle-ci, des sels inorganiques, des polyalcools et des mélange de ces derniers.

9. Comprimé selon la revendication 8, dans lequel le diluant est de la cellulose microcristalline, ses dérivés ou des mélanges de ces derniers.

10. Comprimé selon l'une quelconque des revendications précédentes dans lequel le désintégrant est choisi parmi la polyvinylpyrrolidone réticulée, la croscarmellose de sodium, le carboxyméthyl amidon de sodium, l'amidon, l'amidon prégélatinisé et la cellulose microcristalline.

11. Comprimé selon la revendication 10 dans lequel le désintégrant est la polyvinylpyrrolidone réticulée ou la croscarmellose de sodium

12. Comprimé selon l'une quelconque des revendications précédentes contenant de la silice colloïdale comme agent d'écoulement.

13. Procédé pour la préparation d'un comprimé à avaler selon l'une quelconque des revendications précédentes, qui comprend la granulation humide de la N-acétylcystéine avec un liant et, éventuellement, tout ou partie de la quantité estimée de diluant, le mélange des granulés avec les excipients restants, la compression et le pelliculage éventuel du comprimé.
